(19) Europäisches Patentamt
European Patent Office
Office européen des brevets

(11) Veröffentlichungsnummer: **0 230 627 B1**

(12) # EUROPÄISCHE PATENTSCHRIFT

(45) Veröffentlichungstag der Patentschrift: **04.03.92**

(51) Int. Cl.[5]: **C07D 215/48**, C07D 401/12, C07D 409/12, C07D 405/12, A01N 43/42, A01N 43/48

(21) Anmeldenummer: **86117717.8**

(22) Anmeldetag: **19.12.86**

Die Akte enthält technische Angaben, die nach dem Eingang der Anmeldung eingereicht wurden und die nicht in dieser Patentschrift enthalten sind.

(54) **Oximester von stubstituierten Chinolin-8-carbonsäuren und deren Verwendung als Herbizide.**

(30) Priorität: **23.12.85 DE 3545904**

(43) Veröffentlichungstag der Anmeldung: **05.08.87 Patentblatt 87/32**

(45) Bekanntmachung des Hinweises auf die Patenterteilung: **04.03.92 Patentblatt 92/10**

(84) Benannte Vertragsstaaten: **BE CH DE FR GB IT LI NL**

(56) Entgegenhaltungen:
**EP-A- 0 060 429**
**EP-A- 0 104 389**
**US-A- 3 592 920**

(73) Patentinhaber: **BASF Akfiengesellschaft**
**Carl-Bosch-Strasse 38**
**W-6700 Ludwigshafen(DE)**

(72) Erfinder: **Plath, Peter, Dr.**
**Hans-Balcke-Strasse 13**
**W-6710 Frankenthal(DE)**
Erfinder: **Eicken, Karl, Dr.**
**Am Huettenwingert 12**
**W-6706 Wachenheim(DE)**
Erfinder: **Zeeh, Bernd, Dr.**
**Oueichstrasse 5**
**W-6703 Limburgerhof(DE)**
Erfinder: **Eichenauer, Ulrich, Dr.**
**Paul-Ehrlich-Strasse 25a**
**W-6000 Frankfurt 70(DE)**
Erfinder: **Hagen, Helmut, Dr.**
**Max-Slevogt-Strasse 17e**
**W-6710 Frankenthal(DE)**
Erfinder: **Kohler, Rolf-Dieter, Dr.**
**Amselweg 3**
**W-6803 Edingen-Neckarhausen(DE)**
Erfinder: **Meyer, Norbert, Dr.**
**Dossenheimer Weg 22**
**W-6802 Ladenburg(DE)**
Erfinder: **Wuerzer, Bruno, Dr.**
**Ruedigerstrasse 13**
**W-6701 Otterstadt(DE)**

**EP 0 230 627 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist aus den europäischen Patentveröffentlichungen 60 429 und 104 389 bekannt, daß Ester von herbiziden Chinolin-8-carbonsäuren herbizide Eigenschaften besitzen. Die spezifische Wirkung kann sich jedoch in keinem Fall mit der freien Säure messen.

Die US-A-3 592 920 lehrt, daß beispielsweise Oximester der Benzoesäure und der Zimtsäure herbizid wirksam sind. Ferner ist aus Medicinal Chemistry, A. Burger, Third Edition, Part I, Seiten 73 und 77 bekannt, daß ein heterocyclischer aromatischer Stickstoff (-N=) und eine Methingruppe (-CH=) in bioisosterischen Systemen ausgetauscht werden können.

Es wurde nun gefunden, daß von Chinolin-8-carbonsäuren abgeleitete Oximester der allgemeinen Formel I

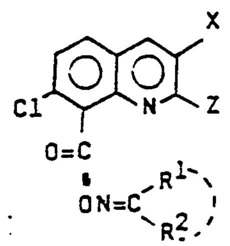

I,

worin

X für Wasserstoff, $C_1$-$C_4$-Alkyl oder Halogen

Z für Wasserstoff oder Methyl steht und worin

$R^1$ bedeutet: Wasserstoff, $C_1$-$C_6$-Alkyl, $C_1$-$C_4$-Alkoxymethyl, $C_1$-$C_4$-Alkoxyethyl, $C_1$-$C_4$-Alkylthiomethyl oder $C_1$-$C_4$Alkylthioethyl, Cyano, $C_1$-$C_4$-Alkoxycarbonyl, $C_3$-$C_6$-Alkenyl oder $C_5$-$C_8$-Cycloalkyl, die jeweils durch Methyl bis zu 3-fach substituiert sein können, Acetyl, Benzoyl, ggf. durch Halogen, Cyan, Trifluoromethyl, $C_1$-$C_4$-Halogenalkyloxy, $C_1$-$C_6$-Alkylthio, $C_1$-$C_4$-Alkoxy, $C_1$-$C_4$-Alkyl, Hydroxy, Dimethylamino oder Acetamino bis zu dreifach substituiertes Benzyl oder Phenyl,

$R^2$ für den Fall, daß $R^1$ von Wasserstoff verschieden ist: Wasserstoff; für den Fall, daß $R^1$ Acetyl bedeutet einen der folgenden Reste $R^3$: $C_1$-$C_6$-Alkyl, $C_1$-$C_4$-Alkoxymethyl, Chlormethyl, Azolylmethyl, 1,1-Dimethoxymethyl, Cyano, $C_1$-$C_4$-Alkoxycarbonyl, $C_1$-$C_4$-Alkoxy, Phenyl;

für den Fall, daß $R^1$ Wasserstoff oder Methyl bedeutet: einen der Reste $R^3$ oder Furyl, Tetrahydrofuryl, Thienyl, Tetrahydropyranyl, Tetrahydrothiopyranyl, Dihydro-$\Delta^3$-pyranyl oder Dihydro-$\Delta^3$-thiopyranyl, oder

$R^1$ und $R^2$ gemeinsam mit dem C-Atom an das sie geknüpft sind:

Cycloalkyliden mit bis zu 12 C-Atomen, $C_5$-$C_6$-Cycloalkenyliden, die jeweils durch Methyl bis zu 3-fach substituiert sein können,

oder den cyclischen Rest eines der folgenden Ketone: Tetrahydrothiopyranon-4, 2,6-Dimethyltetrahydrothiopyran-2-on, 2,6-Dimethyltetrahydropyran-4-on, 2,6-Dimethylpyran-4-on, Tetrahydropyran-4-on, Thiopyran-4-on, 2,6-Dimethylthiopyran-4-on, Tetrahydrofuran-3-on, Benzochinon, Toluchinon, Xylochinon,

eine gegenüber den bisher bekanntgewordenen

Chinolinestern stärkere Herbizidwirkung aufweisen. Außerdem wurde gefunden, daß die Verbindungen gemäß Formel I besser als die zugrundeliegenden Chinolinsäuren geeignet sind, die angestrebte Unkrautbekämpfung im Nachauflaufverfahren zu erreichen, womit die Aufnahme der Wirkstoffe gemäß Formel I über die Pflanzenblätter gemeint ist. Diese Eigenschaft war angesichts der abgeschwächten Wirkung von allen bisher bekanntgewordenen Estern der Chinolincarbonsäuren nicht vorhersehbar und ist als überraschend anzusehen.

Der Ausdruck "$C_1$-$C_6$-Alkyl" umfaßt sowohl geradkettige als auch verzweigte Paraffinkohlenwasserstoffreste mit 1 bis 6 Kohlenstoffatomen wie Methyl, Ethyl, Propyl, Isopropyl, Butyl, Isobutyl, tert.-Butyl, sek.-Amyl, iso-Amyl, n-Pentyl oder n-Hexyl.

Der Ausdruck "$C_1$-$C_4$-Alkoxymethyl" steht für Reste wie Methoxymethyl, Ethoxymethyl, Isopropoxymethyl oder n-Butoxymethyl, während fur $C_1$-$C_4$-Alkoxyethyl etwa 1-Methoxyethyl, 2-Methoxyethyl und 2-Ethoxyethyl. steht.

Der Ausdruck "$C_1$-$C_4$-Alkylthioethyl" umfaßt 2-Methylthioethyl, 1-Methylthioethyl, 2-Isopropylthioethyl, 2-Isobutylthioethyl.

Halogen kann Fluor, Chlor, Brom oder Jod sein. Der Ausdruck "ggf. substituiertes Phenyl" steht z.B. für 4-Fluorphenyl, 2-Chlorphenyl, 3-Chlorphenyl, 4-Chlorphenyl, 4-Bromphenyl, 4-Cyanophenyl, 4-Trifluormethylphenyl, 4-Methoxyphenyl, 3-Methoxyphenyl, 2-Methylphenyl, 3-Methylphenyl, 4-Methylphenyl, 2-Hydroxyphenyl, 4-Dimethylaminophenyl, 4-Acetaminophenyl, 3,4-Dichlorphenyl, 2,4-Dichlorphenyl, 2,6-Dichlorphenyl, 3,5-Dibrom-4-hydroxyphenyl oder 3,5-Dijod-4-hydroxyphenyl.

Der Ausdruck "$C_3$-$C_6$-Alkenyl" umfaßt ungesättigte geradkettige und verzweigte Kohlenwasserstoffreste mit bis zu 6 Kohlenstoffatomen in der Kette, wie z.B. Allyl, Butenyl, Isobutenyl, Pentenyl, Isopentenyl oder Hexenyl.

Der Ausdruck "$C_5$-$C_8$-Cycloalkyl" umfaßt beispielsweise Cyclopentyl, Cyclohexyl, 3,3,5-Trimethylcyclohexyl, Cycloheptyl oder Cyclooctyl.

Mit Azolylmethyl ist Pyrazolylmethyl, Imidazolylmethyl oder Triazolylmethyl gemeint.

Für den Fall, daß $R^1$ und $R^2$ gemeinsam mit den C-Atomen, an das sie geknüpft sind, einen gesättigten oder ungesättigten Ring bilden, der durch Methyl substituiert sein kann und ggf. ein Sauerstoff- oder Schwefelatom im Ring enthalten kann, werden die Oxime folgender Ketone umfaßt: Cyclobutanon, Cyclopentanon, 3-Methylcyclopentanon, 3-Methylcyclopent-2-en-on-1, Cyclohexanon, 2-Methylcyclohexanon, 3-Methylcyclohexanon, 4-Methylcyclohexanon, 3,3,5-Trimethylcyclohexanon, Isophoron, Cyclohex-2-en-on-1, Cycloheptanon, Cyclooctanon, Cyclododecanon, Tetrahydrothiopyranon-4, 2,6-Dimethyltetrahydrothiopyran-4-on, 2,6-Dimethyltetrahydropyran-4-on, 2,6-Dimethylpyran-4-on, Tetrahydropyran-4-on, Thiopyran-4-on, 2,6-Dimethylthiopyran-4-on, Tetrahydrofuran-3-on, Benzochinon, Toluchinon, Xylochinon.

Die erfindungsgemäßen Verbindungen gemäß Formel I werden dadurch erhalten, daß man z.B. ein Säurechlorid der Formel II (dargestellt aus den bekannten Chinolin-8-carbonsäuren gemäß EP 60 429) mit einem Oxim $R^1R^2C=N\text{-}OH$, in einem inerten Lösungsmittel in Gegenwart einer Base bei Temperaturen von -10 °C bis 120 °C, bevorzugt 10 °C bis 50 °C, umsetzt. Als Lösungsmittel eignen sich Pyridin, Toluol, Chlorbenzol, Methylenchlorid, Methyl-tert.-butylether, Tetrahydrofuran. Als Base kann Pyridin, Triethylamin, 1,8-Diazabicyclo[5.4.0]undec-7-on (= DBU), oder bei der Herstellvariante nach Schotten-Baumann, wäßrige Alkalilauge oder Alkalicarbonatlösung dienen.

Die eingesetzten Oxime $R^1R^2C=NOH$ sind bekannte Verbindungen oder können auf den fachüblichen Wegen erhalten werden, die sich aus der jeweiligen Strukturformel ergeben. Bevorzugt wegen des besonders glatten Reaktionsverlaufes sind Oxime von gesättigten (aliphatischen) oder olefinisch ungesättigten Ketonen oder von olefinisch ungesättigten oder aromatischen Aldehyden.

Die Aufarbeitung geschieht bei der Verwendung von Pyridin durch Eingießen in Wasser, bei mit Wasser nicht mischbaren Lösungsmitteln hingegen durch Extraktion mit Wasser, Waschen mit verdünnten Mineralsäuren, Entsäuern, Trocknen und Eindampfen i.V.. Zur Synthese von Estern der Benzaldoxime eignet sich i.a. nur die Pyridin-Variante, da die Produkte in der Wärme oder in Gegenwart starker Säuren zu Benzonitrilen und den bekannten Chinolincarbonsäuren zerfallen.

Die Oxime der Formel III liegen meist in Form von Mischungen ihrer syn- und anti-Isomeren vor. Daher können die Verbindungen der Formel I ebenfalls als syn-anti-Isomerengemische anfallen. Bei Verwendung der Oxime symmetrischer Ketone wie Aceton, Cyclopentanon, Cyclohexanon etc. treten keine Isomeren auf.

Falls eine Reinigung der Rohprodukte erforderlich ist, geschieht dies bei Estern von Ketoximen leicht durch Umkristallisation aus Essigester o.ä., während die Oximester von Benzaldehyden zweckmäßig durch Umfällen aus Ethanol/Wasser oder durch Chromatographie gereinigt werden.

Beispiel 1

Acetonoximester von 3,7-Dichlorchinolin-8-carbonsäure

Zu einer Lösung von 87,6 g (1,2 mol) Acetonoxim in 1,5 l Methylenchlorid gießt man 95 g (1,2 mol) Pyridin und fügt portionsweise unter Eiskühlung 260,5 g (1 mol) 3,7-Dichlorchinolin-8-carbonsäurechlorid zu, so daß die Temperatur zwischen 15 und 20 °C gehalten wird. Nach 8 Stunden Rühren bei 25 °C wird das Reaktionsgemisch zweimal mit 200 ml 5prozentiger HCl ausgeschüttelt und einmal mit Wasser gewaschen. Dann trennt man die organische Phase ab, trocknet über $MgSO_4$ und zieht das Methylenchlorid i.V. ab. Der verbleibende Rückstand wird aus Essigsäureethylester umkristallisiert und i.V. getrocknet. Man erhält 240 g (81 %) eines weißen Pulvers mit Fp. 131 bis 132 °C.

Beispiel 2

Benzaldehydoximester von 3,7-Dichlorchinolin-8-carbonsäure (Verbindung Nr. 16 der Tabelle I)

6,7 g (55 mmol) Benzaldehydoxim werden in 50 ml Pyridin gelöst und bei 0 °C portionsweise mit 13 g (50 mmol) 3,7-Dichlorchinolin-8-carbonsäurechlorid portionsweise versetzt. Nach 3 Stunden Rühren gießt man in ein Gemisch aus 100 ml Eiswasser und 50 ml Eisessig, saugt den ausgefallenen Feststoff ab, rührt ihn mit 150 ml $NaHCO_3$-Wasser durch, saugt wieder ab und wäscht mit Wasser. Nach Trocknen i.V. erhält

man 9 g eines hellbraunen Feststoffs (53 % Ausbeute) mit Fp. 166 bis 168 °C.

Beispiel 3

Cyclohexanoximester von 3,7-Dichlorchinolin-8-carbonsäure (Verbindung Nr. 57 in Tabelle II)

Zu einer eisgekühlten Lösung von 11,3 g (O,1 mol) Cyclohexanonoxim in 1OO ml Pyridin gibt man bei 15 bis 2O °C portionsweise 13 g (5O mmol) 3,7-Dichlorchinolin-8-carbonsäurechlorid. Nach 3 Stunden Rühren wird das Pyridin am Rotationsverdampfer abgezogen, der Rückstand in $CH_2Cl_2$ gelöst, mit Wasser gewaschen, getrocknet ($MgSO_4$) und eingedampft. Der ausgefallene Feststoff wird aus Diisopropylether und Pentan (7:3) umkristallisiert.

Ausbeute: 9,5 g (57 %) weißes Pulver, Fp. 142 bis 143 °C

Unter entsprechender Abwandlung der vorstehenden praktischen Beispiele wurden die übrigen Verbindungen hergestellt, die in den Tabellen I und II zusammengefaßt sind und für die in den Tabellen physikalische Angaben zu ihrer Charakterisierung eingetragen sind. Die nicht mit physikalischen Angaben versehenen Verbindungen können auf entsprechende Weise erhalten werden; sie lassen aufgrund ihrer Struktur als Oximester von herbizid wirkenden Chinolincarbonsäuren ebenfalls eine gute herbizide Wirkung erwarten.

Tabelle I

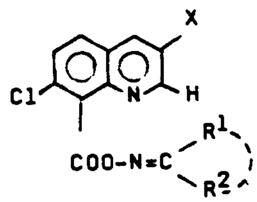

| Nr. | X | $R^1$ | $R^2$ | Fp. (°C) |
|---|---|---|---|---|
| 1 | $CH_3$ | $CH_3$ | $CH_3$ | 111-112 |
| 2 | Cl | $CH_3$ | $CH_3$ | 131-132 |
| 3 | Cl | $C_2H_5$ | $CH_3$ | 130-132 |
| 4 | Cl | $CH_3$ | i-$C_3H_7$ | 144-146 |
| 5 | Cl | n-$C_3H_7$ | n-$C_3H_7$ | 76- 78 |
| 6 | Cl | i-$C_3H_7$ | i-$C_3H_7$ | 103-104 |
| 7 | Cl | i-$C_4H_9$ | i-$C_4H_9$ | 92- 94 |
| 8 | Cl | $CH_3$ | Neopentyl | 133-134 |
| 9 | Cl | $CH_3$ | 4-Methylpentyl | 92- 94 |
| 10 | Cl | $-(CH_2)_2-CH=C(CH_3)_2$ | $CH_3$ | 74- 75 |
| 11 | Cl | $CH_3$ | $CH_2-OCH_3$ | 105-107 |
| 12 | Cl | $C_2H_5$ | $CH_2OCH_3$ | 86- 88 |
| 13 | Cl | $CH_3$ | $CH(OCH_3)_2$ | 117-118 |
| 14 | Cl | $-CH(CH_3)-S-CH_3$ | $CH_3$ | 118-119 |
| 15 | Cl | Benzyl | $CH_3$ | |
| 16 | Cl | H | Phenyl | 166-168 |
| 17 | Cl | $CH_3$ | Phenyl | 173-174 |
| 18 | Cl | Phenyl | $-CH_2Cl$ | |
| 19 | Cl | Phenyl | $-CH_2-$ 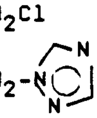 | 185-186 |
| 20 | Cl | Acetyl | $CH_3$ | 114-115 |
| 21 | Cl | Acetyl | Phenyl | 167-169 |
| 22 | Cl | 4-$N(CH_3)_2$-phenyl | H | 165-166 |
| 23 | Cl | 2-Cl-phenyl | H | 158-160 |
| 24 | Cl | 3-Cl-phenyl | H | 155-158 |
| 25 | Cl | 4-Cl-phenyl | H | 175-177 |
| 26 | Cl | 4-F-phenyl | H | 155-157 |
| 27 | Cl | 4-$CF_3$-phenyl | H | 182-183 |
| 28 | Cl | 4-$CH_3$-phenyl | H | 165-167 |
| 29 | Cl | 4-$CH_3O$-phenyl | H | 147-149 |
| 30 | Cl | 3-$CH_3$-phenyl | H | 121-123 |
| 31 | Cl | 3-$CH_3O$-phenyl | H | 141-143 |

Tabelle I (Fortsetzung)

| Nr. | X | $R^1$ | $R^2$ | Fp. (°C) |
|---|---|---|---|---|
| 32 | Cl | 3-($F_2CH-CF_2O$)-phenyl | H | 138-140 |
| 33 | Cl | 4-$H_3CCONH$-phenyl | H | 174-176 |
| 34 | Cl | 4-CN-phenyl | H | 178-180 |
| 35 | Cl | 3,4-$Cl_2$-phenyl | H | |
| 36 | Cl | 2,4-$Cl_2$-phenyl | H | 162-163 |
| 37 | Cl | 2,4-($n-C_6H_{13}-S$)$_2$-phenyl | H | |
| 38 | $CH_3$ | $CH_3$ | $C_2H_5$ | |
| 39 | $CH_3$ | $CH_3$ | $i-C_3H_7$ | |
| 40 | $CH_3$ | $n-C_3H_7$ | $n-C_3H_7$ | |
| 41 | $CH_3$ | $i-C_4H_9$ | $i-C_4H_9$ | |
| 42 | Cl | 2-Thienyl | $CH_3$ | |
| 43 | $CH_3$ | 3-Thienyl | $CH_3$ | |
| 44 | Cl | 2-Furyl | H | |
| 45 | Cl | 2-Furyl | $CH_3$ | 163-164 |
| 46 | Cl | 5,6-Dihydro-$\Delta^3$--thiopyranyl-3 | H | 225-228 |
| 47 | Cl | 5,6-Dihydro-$\Delta^3$-pyranyl-3 | $CH_3$ | 159-161 |
| 48 | Cl | 2-Furyl | $n-C_3H_7$ | |
| 49 | Cl | 2,3-Dihydro-6-methyl-$\Delta^5$-pyran-6-yl | $CH_3$ | |
| 50 | Cl | 4-Hydroxy-3,5-di-bromphenyl | H | 203-205 |
| 51 | Cl | 4-Hydroxy-3,5-di-jodphenyl | H | |
| 52 | Cl | $CH_3$ | $OC_2H_5$ | 129-130 |
| 53 | Cl | CN | $CO_2C_2H_5$ | 138-139 |
| 54 | Cl | 3-Thienyl | $CH_3$ | 160-162 |
| 55 | $CH_3$ | 4-$CF_3$-phenyl | H | 135-137 |
| 56 | $CH_3$ | 2-Cl-phenyl | $CH_3$ | 160-162 |

Tabelle II

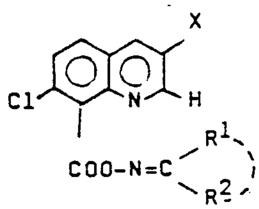

| Nr. | X | 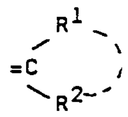 | Fp. (°C) |
|---|---|---|---|
| 57 | Cl | Cyclopentyliden | 128-130 |
| 58 | $CH_3$ | Cyclopentyliden | 105-107 |
| 59 | Cl | Cyclohexyliden | 142-143 |
| 60 | $CH_3$ | Cyclohexyliden | |
| 61 | Cl | 3,3,5-Trimethylcyclohexyliden | 144-145 |
| 62 | Cl | Isophoronyliden 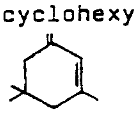 | 140-143 |
| 63 | Cl | 3-Methylcyclopentyliden | 132-133 |
| 64 | Cl | 3-Methylcyclohexyliden | 122-125 |
| 65 | Cl | Cyclopent-2-enyliden | |
| 66 | Cl | Cyclohex-2-enyliden | |
| 67 | Cl | Cycloheptyliden | 120-122 |
| 68 | Cl | 2,6-Dimethylpyranyliden-4 | |
| 69 | Cl | 2,6-Dimethylthiopyranyliden-4 | |
| 70 | Cl | Tetrahydrothiopyranyliden-4 | |
| 71 | Cl | Tetrahydropyranyliden-4 | 167-168 |
| 72 | Cl | Cyclododecyliden | 164-166 |
| 73 | Cl | 2,3-Benzocyclopentyliden-1 | 164-165 |
| 74 | $CH_3$ | 2,3-Benzocyclopentyliden-1 | |
| 75 | Cl | 2,3-Benzocyclohexyliden-1 | 178-179 |
| 76 | $CH_3$ | 2,3-Benzocyclohexyliden-1 | |
| 77 | $CH_3$ | 3,3,5-Trimethylcyclohexyliden | |
| 78 | $CH_3$ | Isophoronyliden | 118-120 |
| 79 | $CH_3$ | 3-Methylcyclopent-2-enyliden | |
| 80 | $CH_3$ | Cycloheptyliden | 108-110 |
| 81 | $CH_3$ | 2,6-Dimethylpyranyliden-4 | |
| 82 | $CH_3$ | Tetrahydropyranyliden-4 | |
| 83 | $CH_3$ | Tetrahydrothiopyranyliden-4 | |

In gleicher Weise sind folgende Verbindungen der Formel I darstellbar:

| Nr. | X | Z | $R^1$ | $R^2$ |
|---|---|---|---|---|
| 84 | $C_2H_5$ | H | $CH_3$ | $CH_3$ |
| 85 | i-$C_3H_7$ | H | $CH_3$ | $CH_3$ |
| 86 | Br | H | $CH_3$ | $CH_3$ |
| 87 | $C_4H_9$ | H | $CH_3$ | $CH_3$ |
| 88 | Cl | $CH_3$ | $CH_3$ | $CH_3$ |
| 89 | H | $CH_3$ | $CH_3$ | $C_2H_5$ |
| 90 | $CH_3$ | H | $CH_2-CH_2-S-CH_3$ | $CH_3$ |
| 91 | $CH_3$ | H | $-CH(CH_3)SCH_3$ | $CH_3$ |
| 92 | $CH_3$ | H | 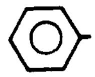$-CH_2$ | $CH_3$ |
| 93 | $CH_3$ | H | 2-Thienyl | $CH_3$ |
| 94 | Cl | H | Phenyl | Pyrazolylmethyl |
| 95 | Cl | H | Phenyl | Imidazolylmethyl |
| 96 | $CH_3$ | H | Phenyl | $-CH_2-Cl$ |
| 97 | $CH_3$ | H | $CH_3$ | $-CH(OCH_3)_2$ |
| 98 | Cl | H | $C_2H_5$ | $C_2H_5$ |
| 99 | Cl | H | n-$C_4H_9$ | n-$C_4H_9$ |
| 100 | Cl | H | iso-$C_5H_{11}$ | $CH_3$ |
| 101 | Cl | H | Allyl | $CH_3$ |
| 102 | Cl | H | $CH_2-CH=CH-CH_3$ | $CH_3$ |
| 103 | Cl | H | Cyclopentyl | $CH_3$ |
| 104 | Cl | H | Cyclohexyl | $CH_3$ |
| 105 | Cl | H | Cyclooctyl | $CH_3$ |
| 106 | Cl | H | 2,6-$Cl_2$-phenyl | H |
| 107 | Cl | H | 2,4,6-$(CH_3)_3$-phenyl | H |
| 108 | $CH_3$ | H | Phenyl | H |
| 109 | $CH_3$ | H | 4-Cl-phenyl | H |

Anwendungsbeispiele

Die Wirkung auf breitblättrige Unkräuter wurde untersucht.

| Lateinischer Name | Deutscher Name | Englischer Name |
|---|---|---|
| Avena sativa | Hafer | oats |
| Galium aparine | Klettenlabkraut | catchweed bedstraw |
| Lamium amplexicaule | Stengelumf. Taubnessel | henbit |
| Triticum aestivum | Weizen | wheat |
| Veronica spp. | Ehrenpreisarten | speedwell |

Bei Nachauflaufapplikation von 3,O kg a.S./ha des Wirkstoffs gemäß Beispiel 1 läßt sich Galium aparine gut bekämpfen. Hafer als Beispielkulturpflanze erleidet keinerlei Schädigung (Tabelle I).

Mit O,5 kg a.S./ha wirkt ein Mittel auf der Grundlage des Beispiels 2 gegen breitblättrige unerwünschte Pflanzen, Weizen bleibt völlig unbeeinflußt. Der Wirkstoff zeigt selektive herbizide Eigenschaften (Tabelle

II).

In Anbetracht des erfaßbaren Wirkungsspektrums zur Unkrautbekämpfung, der Verträglichkeit für Kulturpflanzen oder der erwünschten Beeinflussung des Wachstums derselben sowie angeischts der Vielfalt der Applikationsmethoden können die erfindungsgemäßen Verbindungen in einer großen Zahl von Kulturpflanzen eingesetzt werden.

In Betracht kommen beispielsweise folgende Kulturen:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |
| Camellia sinensis | Teestrauch | tea plants |
| Carthamus tinctorius | Saflor - Färberdistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawn |

| Botanischer Name | Deutscher Name | Englischer Name |
|---|---|---|
| Elaeis guineensis | Ölpalme | oil palms |
| Fragaria vesca | Erdbeere | strawberries |
| Glycine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Erdbohne | mungbeans |
| Phaseolus vulgaris | Buschbohnen | snapbeans, green beans, dry beans |
| Pennisetum glaucum | Perl- oder Rohrkolbenhirse | |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |

| Botanischer Name | Deutscher Name | Englischer Name |
|---|---|---|
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sasamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Zur Verbreiterung des Wirkungsspektrums und zur Erzielung synergistischer Effekte können die neuen Chinolincarbonsäurederivate mit zahlreichen Vertretern anderer herbizider oder wachstumsregulierender Wirkstoffgruppen gemischt und gemeinsam ausgebracht werden.

Außerdem kann es von Nutzen sein, die Verbindungen der Formel I mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Tabelle III - Bekämpfung von Hühnerhirse in Reis bei Vor- und Nachauflaufanwendung neuer Wirkstoffe im Gewächshaus

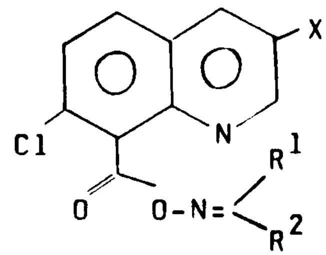

| Beispiel Nr. | X | $R^1$ | $R^2$ | kg a.S./ha | Applikations-art | Testpflanzen und Schädigung (%) Oryzen sativa | Echinochloa crus-galli |
|---|---|---|---|---|---|---|---|
| 20 | Cl | Acetyl | Methyl | 0,25 | VA | 0 | 95 |
| 21 | Cl | Acetyl | Phenyl | 0,5 | VA | 10 | 98 |
| 12 | Cl | Ethyl | Methoxymethyl | 0,5 | VA | 0 | 95 |
| 5 | Cl | Propyl | Propyl | 0,5 | NA | 10 | 90 |
| 20 | Cl | Acetyl | Methyl | 0,125 | NA | 0 | 90 |
| 12 | Cl | Ethyl | Methoxymethyl | 0,25 | NA | 0 | 90 |
| 3 | Cl | Ethyl | Methyl | 0,5 | NA | 10 | 95 |

Zur Bekämpfung von Hühnerhirse, einem bedeutenden Ungras in Reis eignen sich bei Vorauflaufanwendung z.B. die Wirkstoffe 20, 21 und 12 und bei Nachauflaufanwendung die Beispiel Nr. 5, 20, 12 und 3. Die Kulturpflanze wird allenfalls unwesentlich beeinträchtigt.

Tabelle IV - Beispiele zur herbiziden Wirkung und zur Verträglichkeit für eine Beispielkultur bei Nachauflaufanwendung im Gewächshaus

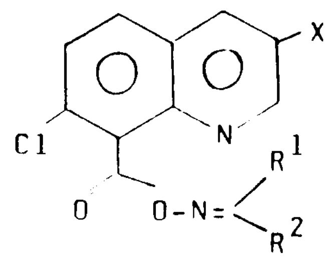

| Beispiel Nr. | X | $R^1$ | $R^2$ | kg a.S./ha | Testpflanzen und Schädigung (%) Triticum aestivum | Galium aparine | Veronica spp. |
|---|---|---|---|---|---|---|---|
| 13 | Cl | Methyl | $CH(OCH_3)_2$ | 0,25 | 10 | 90 | 90 |
| 10 | Cl | $(CH_2CH=C(CH_3)_2)$ | Methyl | 0,5 | 0 | 90 | 98 |
| 20 | Cl | Acetyl | Methyl | 0,5 | 0 | 95 | 98 |
| 16 | Cl | H | Phenyl | 0,5 | 0 | 90 | 98 |

Zur Bekämpfung breitblättriger unerwünschter Pflanzen im Nachauflaufverfahren eignen sich die Wirkstoffe 13, 10, 20 und 16. Weizen als Beispielspflanze für eine Gramineenkultur wird dabei nicht oder nur unwesentlich geschädigt; es handelt sich um selektive heribzide Wirkstoffe.

Tabelle V - Bekämpfung unerwünschten Pflanzenwuchses und Verträglichkeit für eine Beispielkultur bei Vorauflaufanwendung im Gewächshaus

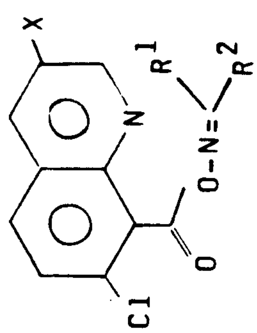

| Beispiel Nr. | X | $R^1$ | $R^2$ | kg a.S./ha | Testpflanzen und Schädigung (%) Triticum aestivum | Echinochloa c.g. | Galium aparine | Veronica spp. |
|---|---|---|---|---|---|---|---|---|
| 13 | Cl | Methyl | $CH(OCH_3)_2$ | 0,5 | 10 | 98 | 90 | 100 |
| 20 | Cl | Acetyl | Methyl | 0,25 | 10 | 98 | 90 | 100 |

Bei hoher Verträglichkeit für die Kulturpflanze Weizen lassen sich mit den Wirkstoffen 13 und 20 grasartige und breitblättrige unerwünschte Pflanzen gut bekämpfen.

## Patentansprüche

**1.** Oximester von substituierten Chinolin-8-carbonsäuren gemäß Formel I

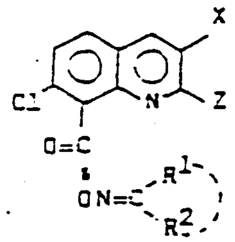

I,

worin
X für Wasserstoff, $C_1$-$C_4$-Alkyl oder Halogen
Z für Wasserstoff oder Methyl steht und worin
$R^1$ bedeutet: Wasserstoff, $C_1$-$C_6$-Alkyl, $C_1$-$C_4$-Alkoxymethyl, $C_1$-$C_4$–Alkoxyethyl, $C_1$-$C_4$-Alkylthiomethyl oder $C_1$-$C_4$Alkylthioethyl, Cyano, $C_1$-$C_4$-Alkoxycarbonyl, $C_3$-$C_6$-Alkenyl oder $C_5$-$C_8$-Cycloalkyl, die jeweils durch Methyl bis zu 3-fach substituiert sein können, Acetyl, Benzoyl, ggf. durch Halogen, Cyan, Trifluormethyl, $C_1$-$C_4$-Halogenalkyloxy, $C_1$-$C_6$-Alkylthio, $C_1$-$C_4$-Alkoxy, $C_1$-$C_4$-Alkyl, Hydroxy, Dimethylamino oder Acetamino bis zu dreifach substituiertes Benzyl oder Phenyl,
$R^2$ für den Fall, daß $R^1$ von Wasserstoff verschieden ist: Wasserstoff; für den Fall, daß $R^1$ Acetyl bedeutet einen der folgenden Reste $R^3$: $C_1$-$C_6$-Alkyl, $C_1$-$C_4$-Alkoxymethyl, Chlormethyl, Azolylmethyl, 1,1-Dimethoxymethyl, Cyano, $C_1$-$C_4$-Alkoxycarbonyl, $C_1$-$C_4$-Alkoxy, Phenyl;
für den Fall, dap $R^1$ Wasserstoff oder Methyl bedeutet: einen der Reste $R^3$ oder Furyl, Tetrahydrofuryl, Thienyl, Tetrahydropyranyl, Tetrahydrothiopyranyl, Dihydro-$\Delta^3$-pyranyl oder Dihydro-$\Delta^3$-thiopyranyl, oder
$R^1$ und $R^2$ gemeinsam mit dem C-Atom an das sie geknüpft sind:
Cycloalkyliden mit bis zu 12 C-Atomen, $C_5$-$C_6$-Cycloalkenyliden, die jeweils durch Methyl bis zu 3-fach substituiert sein können
oder den cyclischen Rest eines der folgenden Ketone:
Tetrahydrothiopyranon-4, 2,6-Dimethyltetrahydrothiopyran-4-on, 2,6-Dimethyltetrahydropyran-4-on, 2-6-Dimethylpyran-4-on, Tetrahydropyran-4-on, Thiopyran-4-on, 2,6-Dimethylthiopyran-4-on, Tetrahydrofuran-3-on, Benzochinon, Toluchinon, Xylochinon.

**2.** Herbizides Mittel, dadurch gekannzeichnet, daß es eine wirksame Menge einer Verbindung gemäß Formel I enthält.

**3.** Verfahren zur Herstellung von Verbindungen der Formel I, dadurch gekennzeichnet, daß man ein entsprechendes Säurehalogenid der Formel II

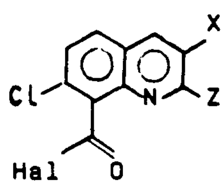

II

mit einem entsprechenden Oxim der Formel III

$R^1R^2C=NOH$ III

umsetzt.

**Claims**

**1.** An oxime ester of a substituted quinoline-8-carboxylic acid of the formula I

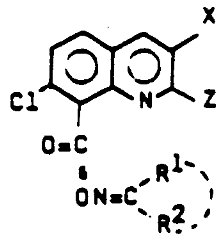

I,

where X is hydrogen, $C_1$-$C_4$-alkyl or halogen, Z is hydrogen or methyl, $R^1$ is hydrogen, $C_1$-$C_6$-alkyl, $C_1$-$C_4$-alkoxymethyl, $C_1$-$C_4$-alkoxyethyl, $C_1$-$C_4$-alkylthiomethyl or $C_1$-$C_4$-alkylthioethyl, cyano, $C_1$-$C_4$-alkoxycarbonyl, $C_3$-$C_6$-alkenyl or $C_5$-$C_8$-cycloalkyl, each of which bears up to 3 methyl substituents, acetyl, benzoyl, unsubstituted benzyl or phenyl, or benzyl or phenyl bearing up to 3 substituents selected from the group consisting of halogen, cyano, trifluromethyl, $C_1$-$C_4$-haloalkyloxy, $C_1$-$C_6$-alkylthio, $C_1$-$C_4$-alkoxy, $C_1$-$C_4$-alkyl, hydroxy, dimethylamino or acetamino, $R^2$ is hydrogen if $R^1$ is not hydrogen, $R^2$ further is one of the following radicals $R^3$ from the group consisting of $C_1$-$C_6$-alkyl, $C_1$-$C_4$-alkoxymethyl, chloromethyl, azolylmethyl, 1,1-dimethoxymethyl, cyano, $C_1$-$C_4$-alkoxycarbonyl, $C_1$-$C_4$-alkoxy or phenyl if $R^1$ is acetyl, or $R^2$ is, when $R^1$ is hydrogen or methyl, one of the radicals $R^3$ or furyl, tetrahydrofuryl, thienyl, tetrahydropyranyl, tetrahydrothiopyranyl, dihydro-$\Delta^3$-pyranyl or dihydro-$\Delta^3$-thiopyranyl; or $R^1$ and $R^2$, together with the carbon atom to which they are linked, are cycloalkylidene having up to 12 carbon atoms or $C_5$-$C_6$-cycloalkenylidene each of which bears up to 3 methyl substituents, or the cyclic radical of one of the following ketones: tetrahydrothiopyran-4-one, 2,6-dimethyltetrahydrothiopyran-4-one, 2,6-dimethyltetrahydropyran-4-one, 2,6-dimethylpyran-4-one, tetrahydropyran-4-one, thiopyran-4-one, 2,6-dimethylthiopyran-4-one, tetrahydrofuran-3-one, benzoquinone, toluquinone or xyloquinone.

**2.** A herbicidal composition containing an effective amount of a compound of the formula I.

**3.** A process for the preparation of compounds of the formula I, wherein an appropriate acid halide of the formula II

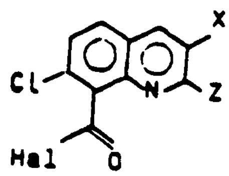

II

is reacted with an approximate oxime of the formula III

$R^1R^2C = NOH$ III.

**Revendications**

**1.** Esters d'oximes et d'acides quinoléine-8-carboxyliques substitués, selon la formule I

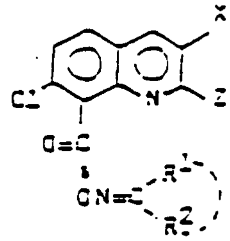

I,

dans laquelle

X est mis pour hydrogène, alkyle en C 1-C 4 ou halogène,

Z est mis pour hydrogène ou méthyle,

$R^1$ représente hydrogène, alkyle en C 1-C 6, alcoxy (C 1-C 4) méthyle, alcoxy (C 1-C 4) éthyle, alkyl (C 1-C 4) thiométhyle ou alkyl (C 1-C 4) thioéthyle, cyano, alcoxy (C 1-C 4) carbonyle, alcényle en C 3-C 6 ou cycloalkyle en C 5-C 8, qui peuvent, chacun, être substitués jusqu'à trois fois par méthyle; acétyle, benzoyle; benzyle ou phényle, éventuellement substitués jusqu'à trois fois par halogène, cyano, trifluorométhyle, halogénalkyl (C 1-C 4) oxy, alkyl (C 1-C 6) thio, alcoxy en C 1-C 4, alkyle en C 1-C 4, hydroxy, diméthylamino ou acétamino,

$R^2$, dans le cas où $R^1$ ne représente pas hydrogène : hydrogène; dans le cas où $R^1$ représente acétyle, l'un des restes $R^3$ suivants :

alkyle en C 1-C 6, alcoxy-(C 1-C 4) méthyle, chlorométhyle, azolyl = méthyle, 1,1-diméthoxyméthyle, cyano, alcoxy (C 1-C 4) carbonyle, alcoxy en C 1-C 4, phényle;

dans le cas où $R^1$ représente hydrogène ou méthyle : un des restes $R^3$ ou furyle, tétrahydrofuryle, thiényle, tétrahydropyranyle, tétrahydrothiopyranyle, dihydro-$\Delta^3$-pyranyle ou dihydro-$\Delta^3$-thiopyranyle, ou

$R^1$ et $R^2$, simultanément avec l'atome C auquel ils sont reliés : cycloalkylidène ayant jusqu'à 12 atomes C, cycloalcénylidène en C 5 ou C 6, pouvant être substitués chacun jusqu'à trois fois par méthyle,

ou le reste cyclique d'une des cétones suivantes :

tétrahydrothiopyranone-4, 2,6-diméthyltétrahydrothiopyran-4-one, 2,6-diméthyltétrahydropyran-4-one, tétrahydropyran-4-one, thiopyran-4-one, 2,6-diméthylthiopyran-4-one, tétrahydrofuran-3-one, benzoquinone, toluquinone, xyloquinone.

**2.** Herbicide, caractérisé par le fait qu'il contient une quantité efficace d'un composé selon la formule I.

**3.** Procédé de préparation de composés, de la formule I, caractérisé par le fait que l'on fait réagir un halogénure d'acide approprié, de formule II

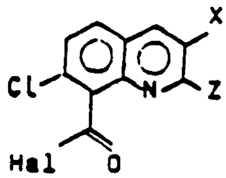

II

avec une oxime appropriée de formule III

$R^1R^2C = NOH$ III